# EUROPEAN PATENT APPLICATION

(11) **EP 3 915 600 A1**
(43) Date of publication of application: **01.12.2021**
(21) Application number: 20176258.0
(22) Date of filing: 25.05.2020
(51) Int. Cl.: A61M 1/00

(54) **A NEGATIVE PRESSURE WOUND THERAPY (NPWT) SYSTEM**

(71) Applicant: Mölnlycke Health Care AB, 402 52 Göteborg (SE)
(72) Inventor: ROUX, Alain, 432 74 TRÄSLÖVSLÄGE (SE); SVENSSON, Malin, 414 53 GÖTEBORG (SE); SKEPPSTEDT, Viktoria, 426 58 VÄSTRA FRÖLUNDA (SE); HOLMÉN, Malin, 412 66 GÖTEBORG (SE); BOLYOS, Elinor, 438 91 LANDVETTER (SE)
(74) Representative: Kransell & Wennborg KB

(57) **Abstract**

The present disclosure generally relates to a negative wound therapy (NPWT) system (100) comprising a mobile negative pressure device (101), a tubing assembly (105) and a non-absorbent or an absorbent dressing (104).

## Description

### TECHNICAL FIELD

The present disclosure generally relates to a negative pressure wound therapy (NPWT) system comprising a mobile negative pressure device, a tubing assembly and a non-absorbent or an absorbent dressing

### BACKGROUND

Negative pressure wound therapy (NPWT) is a technique that promotes healing of e.g. surgical, acute and chronic wounds by the application of a sub-atmospheric pressure to the wound, using a negative pressure pump. Wound healing is achieved by applying a negative pressure, such as vacuum through a dressing or a cover applied onto the wound. Excess wound exudate is thereby drawn out, which increases the blood flow to the area, and promotes the formation of granulation tissue. The NPWT technique also permits less outside disturbance of the wound and transports excess fluids away from the wound site.

The NPWT technique has, until now, mainly been applied to a patient while in a hospital environment. However, recent product development allows the technique to be used by a patient in a home environment.

In a hospital setting, the wound to be treated is typically an open cavity wound, which is first filled with a wound filler, such as a gauze or a foam. The wound may thereafter be sealed with an adhesive film, and connected to a negative pressure pump via a drain or a port. The size of the foam, gauze and/or the adhesive film may be adapted and cut depending on the size, shape or type of wound. The application procedure is typically carried out by a caregiver. The negative pressure pump used in such a system is typically of a large size and generally has a high capacity to be able to deal with large amounts of wound exudate.

In a home environment, a portable NPWT device, which may be carried around by the patient, is generally preferred. A portable NPWT device typically comprises an absorbent dressing configured to be connected to a negative pressure source by means of a tubing. The pump used is in such devices is typically of a smaller size, and has a more limited capacity. An NPWT system comprising an absorbent dressing is also utilized in a hospital or care facility, particularly on less exuding or "closed" wounds, such as surgically closed incisions.

In most portable NPWT systems, the dressing serves as the sole means to collect wound exudate, whereas in NPWT systems adapted for the treatment of open cavity wounds (in a hospital setting), a fluid collection means, such as a canister, arranged remote from the wound site, is included. In such a system, the canister serves as the predominant means for collection of wound exudate.

Regardless of whether the NPWT system comprises a non-absorbent or an absorbent dressing or whether the application procedure is to be carried out by a caregiver or a wearer in his/her home environment, there is room for improvements in this field, particularly with respect to relieving the burden for caregivers and patients dealing with such wounds. Not only does an exuding wound cause great discomfort and/or pain to the affected person, but it also causes difficulties to nursing personnel and other caregivers. The system components typically vary largely between an NPWT system that is aimed at low exuding, and high exuding wounds, respectively, and various mechanisms and algorithms of the pump typically need to be adjusted or changed depending on the dressing and the system components utilized.

There is therefore a need to provide a simple and reliable NPWT system that can be used for all types of wounds and all types of wearers or patients, in any care situation or care setting.

### SUMMARY

In view of the above-mentioned problems, it is an object of the present disclosure to provide improvements with respect to NPWT systems, particularly with respect to providing a system being simple and suitable for use for all types of wounds.

According to a first aspect, there is provided a negative pressure wound therapy (NPWT) system comprising
- a mobile negative pressure wound therapy (NPWT) device comprising a housing, a negative pressure pump arranged within the housing and a canister detachably connected to the housing,
- an absorbent or a non-absorbent dressing,
- a tubing assembly configured to fluidly connect the absorbent or non-absorbent dressing to the NPWT device, wherein the tubing assembly comprises a
   - a first tubing configured to be connected to the dressing, wherein a distal end of the first tubing is attached to a first connector portion;
   - a second tubing configured to be connected to the canister, wherein a distal end of the second tubing is attached to a second connector portion configured to be detachably connected to the first connector portion, wherein the system comprises means to supply air to the dressing at a rate of from 2 to 7 ml/min during operation.

The present disclosure is based on the realization that a supply of air within the range of from 2 to 7 ml/min allows for the negative pressure wound therapy device and the remaining components of the NPWT system to be used with both non-absorbent and absorbent dressings. There is no need to replace or change the system set-up, but the same setting can be utilized for both scenarios. In other words, the NPWT system is suitable for use with all types of wounds, both high and low exuding wounds.

For example, in a hospital setting, the nursing personnel may examine the wound and determine that a non-absorbent dressing (or an absorbent dressing) is the best treatment option. Instead of applying a completely different set of components; i.e. a different kind of negative pressure source, applying a separate canister etc. or adapting the mechanisms or algorithms of the negative pressure pump, the same types of components can be utilized and only the dressing type needs to be varied.

Furthermore, the system is easy to assemble, and each component can easily and quickly be connected and disconnected. For example, the wearer may easily disconnect the NPWT dressing from the NPWT device if he/she is to take a shower or change clothes.

Since the first connector portion of the dressing tubing is the same regardless of whether the dressing is absorbent or non-absorbent, the second connector portion of the second (canister) tubing is configured to be connected to both dressing types. This is beneficial as it allows for flexibility and ease of use for a caregiver. For example, the caregiver may, upon checking the wound, come to the conclusion that the characteristics of the wound have changed and that a different type of dressing (for example a non-absorbent instead of an absorbent dressing or vice versa), and thereby easily disconnect the NPWT device from the dressing, replace the dressing and re-connect the NPWT device with the replaced dressing.

Supplying air to a NPWT system is known in the context of open cavity wounds and adhesive film dressings, particularly for the purpose of resolving potential exudate blockages or liquid columns formed in the tubing. The present inventors have found that a rate of from 2 to 7 ml/min is also beneficial for less exuding wounds and has a positive impact when the NPWT system is used in conjunction with absorbent dressings. Furthermore, this range represents an interval which can be utilized for both system set-ups.

If too much air is introduced, this may negatively impact the stability of the system, and the pump must typically be activated on a higher frequency.

In embodiments, the means to supply air to the dressing is configured to supply air at a rate of from 2 to 7 ml, preferably from 3 to 5 ml/min, at a negative pressure of from -80 to -180 mmHg, preferably from -100 to -150 mmHg, more preferably from -110 to - 140 mmHg.

The means to supply air to the dressing may involve allowing ambient air to enter the system in a controlled manner. For example, ambient air may be introduced into the system by an inlet associated with the first and/or the second connector portion. The NPWT system is not limited to a particular means to supply air, but any means can be used as long as the rate of air is controlled and maintained at a level of from 2 to 7 ml/min during operation, e.g. from 3 to 5 ml/min during operation.

In embodiments, the NPWT device is configured to regulate the supply of air to the dressing.

In embodiments, the means to supply air comprises an air filter arranged in the first and/or the second connector portion, wherein the air filter is configured to control the supply of air into the dressing and/or the first tubing.

This arrangement is particularly preferred if the means to supply air to the dressing is by means of the introduction of ambient air in the connector portion(s). The air filter may be arranged in an air inlet associated with the first and/or the second connector portion.

The air filter preferably comprises a hydrophobic and porous material, wherein the size of the pores is within the range of from 2 to 20 µm, preferably in the range of from 5 to 12 µm.

The air filter may comprise polyethylene, preferably sintered polyethylene.

Such a filter is associated with improved filtering characteristics, a good chemical resistance and thermoplasticity, and is also environmentally friendly.

In embodiments, the first tubing comprises a fluid conduit configured to remove fluid from the dressing and an air conduit configured to supply air to the dressing and/or the fluid conduit.

This allows for a smooth and even supply of air into the dressing, and is beneficial in resolving potential liquid column and liquid obstruction problems in the tubing.

In embodiments, the non-absorbent or absorbent dressing comprises at least a backing layer comprising a thermoplastic elastomer and an adhesive skin contact layer comprising a silicone gel.

A backing layer comprising a thermoplastic elastomer is pliable and is able to be stretched to moderate elongations, and upon the removal of stress, return to its original shape. The adhesive skin contact layer is arranged in contact with the skin or the wound of a wearer, and a skin friendly silicone gel is therefore preferred. The silicone adhesive skin contact layer is sufficiently adherent to skin such that the dressing stays in place, yet is configured to maintain its adherence with repeated removal and re-application.

Preferably, the adhesive skin contact layer is a laminate comprising a polyurethane film and a silicone gel layer.

The polyurethane film provides rigidity and stability to the backing layer, but still allows for the adhesive skin contact layer to be pliable and adhere and stay on the skin of the wearer during movement.

In embodiments, the backing layer of the dressing has a moisture vapor transmission rate (MVTR) in the range of from 500 to 3500 g/m2/24h, preferably in the range of from 700 to 2600 g/m2/24h as measured by NWSP070.4R0(15).

An MVTR in the above range provides various advantages to the NPWT system and the applied therapy. The inventors have found that with a relatively low MVTR of the backing layer, a more stable therapy with less frequent activations of the negative pressure pump, is observed. Overall, this has positive effects in terms of battery consumption, reduction of noise and a prolonged and more stable wound therapy. This is particularly surprising when the system comprises an absorbent dressing, which typically comprises a backing layer having a significantly higher moisture vapor transmission rate.

In embodiments, the dressing is absorbent and the first tubing is pre-attached to the dressing.

The tubing may be attached by means of a coupling member connected to the dressing. The coupling member is attached to the backing layer of the dressing. The fact that the first tubing is pre-attached allows for a quick assembly of the components of the system. This way, the wearer can quickly and easily disconnect and re-connect the dressing from the NPWT device.

In embodiments, the dressing is absorbent and comprises a backing layer, an adhesive skin contact layer and an absorbent structure arranged between the adhesive skin contact layer and the backing layer, wherein the backing layer and the adhesive skin contact layer are configured to extend beyond the periphery of the absorbent structure to form a border portion along the contour of the absorbent structure.

In other words the absorbent dressing is a so called "bordered dressing".

In embodiments, the absorbent dressing comprises a transmission layer arranged between the backing layer and the absorbent structure, wherein the transmission layer comprises a spacer fabric.

The transmission layer serves to facilitate the transmission of negative pressure from the negative pressure source to the wound site.

In embodiments, the dressing has a retention capacity of from of from 300 to 700 mg/cm2, preferably from 400 to 600 mg/cm2, as measured by the test method described hereinafter.

The inventors have found that the retention capacity of the dressing largely influences the distribution of exudate between the dressing and the canister. In an NPWT system of the present disclosure (wherein the dressing is absorbent), both the dressing and the canister are configured to manage excess wound exudate. An appropriate balance between the distribution of wound exudate stored by the dressing, and the canister, respectively, is thus desirable. For example, if the dressing absorbs too fast, and too "much", the dressing may become saturated before the exudate transfer towards the canister has initiated. This may negatively affect the dressing's ability to stay on the skin; i.e. the wear time of the dressing is reduced. An improper balance between the canister and the dressing results, and the dressing typically needs to be changed relatively often. In contrast, if too much exudate is transferred to the canister, the canister becomes the predominant means for fluid collection. This is also undesirable since a too quick flow of exudate may obstruct the port and conduits connecting the canister with the dressing, and the transmission of negative pressure to the wound site may be impaired. Furthermore, the canister may need to be replaced and emptied more frequently, and the full absorbent capacity of the dressing is thus not utilized.

The dressing is therefore tailored to provide a balanced distribution between wound exudate absorbed and handled by the dressing, and wound exudate being removed therefrom; i.e. to the canister.

In embodiments, the absorbent dressing comprises a liquid spreading layer between the absorbent structure and the backing layer.

The provision of a liquid spreading layer between the absorbent structure and the backing layer provides several advantages in terms of liquid handling and liquid distribution. The liquid spreading layer allows for a controlled and balanced liquid distribution between the dressing and the canister. The dressing's ability to function as a fluid collection means is optimized, while still allowing for the removal and transport of a substantial portion of exudate from the dressing by means of the tubing. Furthermore, the liquid spreading layer improves the spreading and distribution of wound exudate within the dressing, thereby forming a larger surface area from which exudate can evaporate from the dressing (through the backing layer). The larger surface area of the liquid spreading layer may thus act to more efficiently get rid of excess exudate and keep the wound site relatively dry.

The liquid spreading layer also improves the distribution of potential "backflow" exudate; i.e. exudate flowing in the opposite direction (from the tubing to the dressing). This may for example occur if the dressing is disconnected from the negative pressure source and/or the remote fluid collection means. The liquid spreading layer secures that such back-flow of exudate is spread out rather than flowing back towards the wound site in one spot. This way, the wound site can be kept relatively dry.

Typically, the backing layer comprises a coupling member configured to connect the dressing to the first tubing and wherein the backing layer and at least a portion of the absorbent structure comprises an opening; the opening being arranged underneath the coupling member, wherein the liquid spreading layer, if present, is void of an opening.

The opening serves to secure fluid communication between the wound site and the tubing of the dressing; and thereby also fluid communication between the wound site and the canister. The liquid spreading layer, where present, is void of such an opening to prevent potential gelling particles and undesired larger particulate of the exudate from entering the tubing of the dressing. In the area underlying the coupling member of the dressing, the liquid spreading layer is configured to transfer liquid from within the dressing through the tubing and to the canister.

According to another aspect of the present disclosure, there is provided a negative pressure wound therapy (NPWT) system, the NPWT system comprising a wound dressing, the wound dressing provided for creating a sealed space defined in part by a wound site, the wound dressing being at least one of an absorbent or a non-absorbent dressing, the non-absorbent or absorbent dressing comprises at least a backing layer comprising a thermoplastic elastomer and an adhesive skin contact layer comprising a silicone gel, means for supplying air to the wound dressing at a predetermined supply rate, the rate being from 2 to 7 ml/min, preferably from 3 to 5 ml/min and an NPWT device, comprising a housing, a battery arranged within the housing, a negative pressure pump arranged within the housing, a control unit arranged within the housing, the control unit being electrically connected to the battery and the negative pressure pump, and a canister fluidly coupled to the negative pressure pump and to the wound dressing, wherein the control unit is arranged to determine an intermediate voltage level of the battery, select a pulse width modulation (PWM) scheme for operating the negative pressure pump, the PWM scheme selected based on the predetermined supply rate, the intermediate voltage level and a predetermined level of negative pressure, wherein the predetermined level of negative pressure is from -80 to -180 mmHg, preferably from -100 to -150 mmHg, more preferably from -110 to -140 mmHg, and operate the negative pressure pump according to the selected PWM scheme.

In line with the present aspect of the present disclosure, the control unit comprised with the NPWT device has been adapted to ensure that the negative pressure provided at the sealed space is within a predetermined range, independent on what type of wound dressing that has been connected to the NPWT device.

This functionality is achieved by closely monitoring fixed and changing operational parameters for the NPWT device, taking into account an expected leakage and a desired negative pressure, while balancing this with an intermediate voltage level of the battery. Specifically, the intermediate voltage level of the battery will change over time of operation, where typically the intermediate voltage level of the battery will decrease while operating the NPWT device.

Furthermore, it is in line with the present disclosure desirable to ensure that the operational speed of the negative pressure pump is kept essentially constant (such as within exemplary +/- 10% while the negative pressure pump is active), since keeping the speed of the negative pressure pump essentially constant will also result in a sound level of the negative pressure pump at an essentially constant level, reducing a disturbance of the patient using the NPWT device.

The NPWT system as described hereinbefore may be provided as a kit of components, wherein the kit comprises both a non-absorbent and an absorbent dressing.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF THE DRAWINGS

The various aspects of the present disclosure, including its particular features and advantages, will be readily understood from the following detailed description and the accompanying drawings, in which:
Figure 1 conceptually illustrates a negative pressure wound treatment (NPWT) system according to an exemplary embodiment of the present disclosure.
Figure 2 illustrates the system in the form of a kit of components, wherein the kit comprises an absorbent dressing.
Figure 3 illustrates the system in the form of a kit of components, wherein the kit comprises a non-absorbent dressing.
Figure 4a illustrates an absorbent dressing suitable for use in the NPWT system of the present disclosure.
Figure 4b illustrates a cross-sectional view of an absorbent dressing suitable for use in the NPWT system of the present disclosure.
Figure 4c illustrates a split view of an absorbent dressing suitable for use in the NPWT system of the present disclosure dressing.
Figure 5 illustrates the average Toff as measured with a system of the present disclosure when using a dressing according to an exemplary embodiment having an MVTR of 2530 g/m2/24h compared to a reference dressing having an MVTR of 3940 g/m²/24h.
Figure 6 illustrates the liquid distribution between the canister and an absorbent dressing according to an exemplary embodiment and two reference dressings when used in a system of the present disclosure.
Figure 7a is a picture of an absorbent reference dressing after a test period of 7 days with the NPWT system of the present disclosure.
Figure 7b is a picture of another absorbent reference dressing after a test period of 7 days with the NPWT system of the present disclosure.
Figure 7c is a picture of an absorbent dressing according to an exemplary embodiment of the present disclosure after a test period of 9 days with the NPWT system of the present disclosure.
Figure 8a illustrates pictures of an absorbent dressing according to an exemplary embodiment of the present disclosure compared to a reference dressing, after exposure to liquid, as seen from the backing layer of the dressings.
Figure 8b illustrates pictures of an absorbent dressing according to an exemplary embodiment of the present disclosure compared to a reference dressing, after exposure to liquid, as seen from the transmission layer, when the adhesive skin contact layer has been removed.
Figure 9 present an exemplary functional scheme of a NPWT device according to an exemplary embodiment of the present disclosure.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like reference characters refer to like elements throughout.

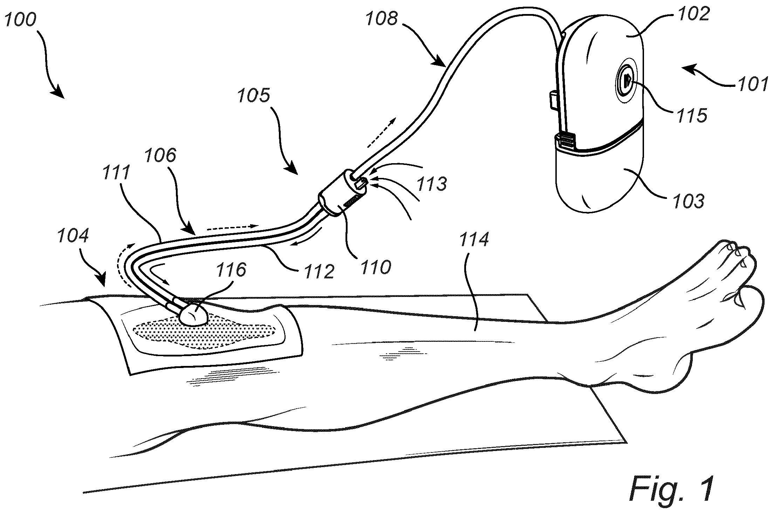

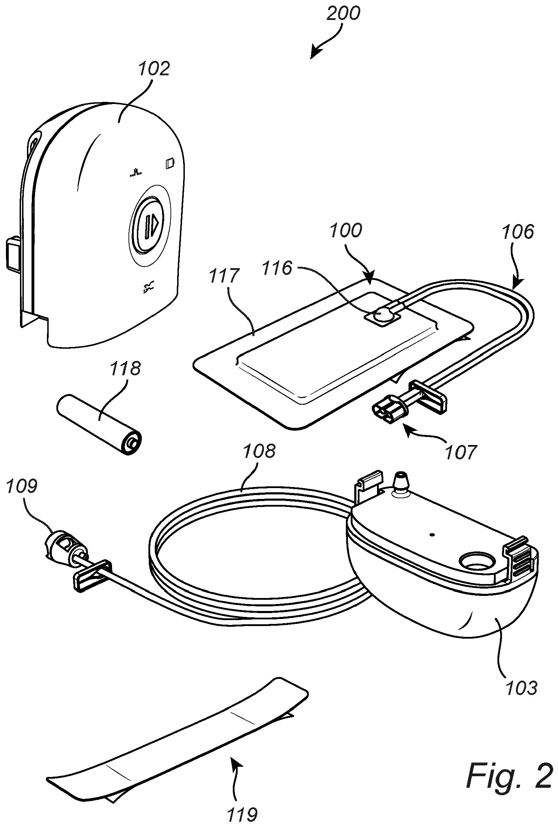

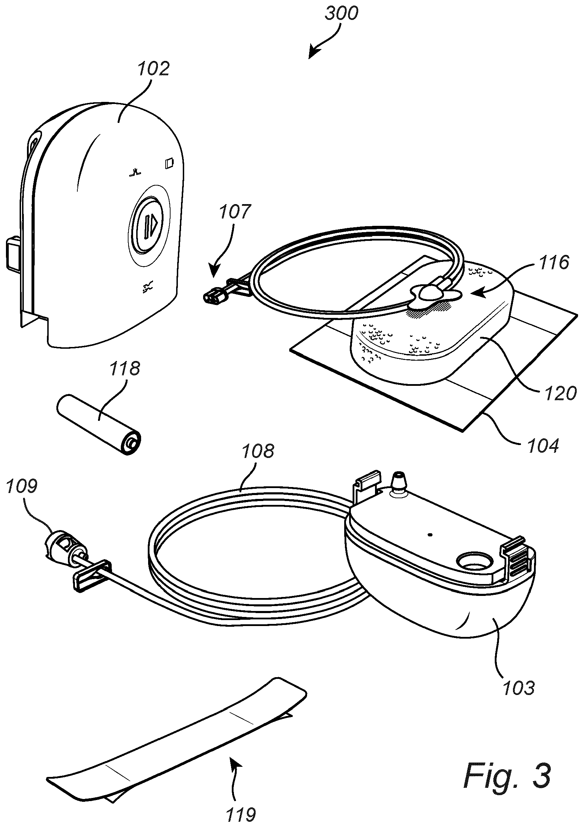

With reference to figure 1, a negative pressure wound therapy (NPWT) system according to the present disclosure is conceptually illustrated. The NPWT system 100 comprises:
- a negative pressure wound therapy (NPWT) device 101 comprising a housing 102, a negative pressure pump arranged within the housing 102 and a canister 103 detachably connected to the housing 102,
- an absorbent or a non-absorbent dressing 104,
- a tubing assembly 105 configured to fluidly connect the absorbent or non-absorbent dressing 104 to the NPWT device 101, wherein the tubing assembly 105 comprises:
   - a first tubing 106 configured to be connected to the dressing 104, wherein a distal end of the first tubing 106 is attached to a first connector portion (see 107 in figure 2 or figure 3);
   - a second tubing 108 configured to be connected to the canister 103, wherein a distal end of the second tubing 108 is attached to a second connector portion (see 109 in figure 2 or figure 3) configured to be detachably connected to the first connector portion, wherein the system 100 comprises means to supply air to the dressing 104 at a rate of from 2 to 7 ml/min during operation.

Preferably, the means to supply air to the dressing is configured to supply air at a rate of from 2 to 7 ml, preferably from 3 to 5 ml/min, at a negative pressure of from -80 to -180 mmHg, preferably from -100 to -150 mmHg, more preferably from -110 to -140 mmHg.

In the context of the present disclosure, "negative pressure wound therapy" refers to a therapy utilizing a source of negative pressure (e.g. a vacuum pump) to remove excess fluid from a wound. The wound may be an open wound or it may be a closed wound; i.e. a surgically closed incision, and the term therefore also encompasses "topical negative pressure (TNP) therapy" applications, which is a term often used in the context of closed incisions.

As used herein, the term "fluidly connected" should be interpreted broadly and may comprise e.g. any form of tubing, conduits, or channels providing a fluid connection/communication between the canister 103 and the negative pressure pump and the dressing 104.

The negative pressure pump is adapted for establishing a negative pressure when the negative pressure pump is in an active state. The negative pressure pump may be any type of pump that is biocompatible and maintains or draws adequate and therapeutic vacuum levels. In embodiments, the negative pressure pump is a pump of the diaphragmatic or peristaltic type. The negative pressure level to be achieved is in a range between about -80 to -180 mmHg, preferably from -100 to -150 mmHg, more preferably from -110 to -140 mmHg.

The canister 103 is detachably connected to the housing 102. In other words, the canister 103 is releasably connected to the housing. The detachable connection may be by conventional means including a friction fit, bayonet coupling, snap fit, barbed connector, or the like. The detachable configuration allows the user or caregiver to remove the canister 103 and empty the collected liquid, and subsequently re-attach the canister 103 to the housing 102 again.

The canister 103 may be formed from e.g. molded plastic or the like. The canister 103 is preferably at least partly transparent/translucent to permit viewing into the interior of the canister 103 to assist the user in determining the remaining capacity of the canister 103.

For example, an inner volume of the canister 103 is between 30 - 200 ml, e.g between 40 and 100 ml. The inner volume of the canister 103 may vary depending on the type of wound. In embodiments, the canister 103 comprises a liquid absorbent material. In a possible embodiment at least 75% of the inner volume of the canister 103 is occupied with a liquid absorbent material.

The negative pressure pump is fluidly connected to the canister 103. An inlet port may be formed at the canister 103 to allow connection to the second tubing 108. An inlet portion may also be formed elsewhere at the NPWT device 101, however still fluidly connected to the canister 103. The connection between the inlet port and the second tubing 108 is a sealed connection to ensure that no leakage is formed during normal operation of the NPWT device 101. The second tubing 108 may be releasably connected to the inlet port through conventional means including a friction fit, bayonet coupling, snap fit, barbed connector, or the like.

The NPWT device 101 is connected to the dressing 104 by means of the tubing assembly 105. In figure 1, the first connector portion 107 and the second connector portion 109 are illustrated in a connected state to form a connector unit 110. The connector unit 110 is arranged at a position between the dressing 104 and the NPWT device 101. The first and the second connector portions 107 and 109 are detachably connected such that the dressing 104 can easily be disconnected from the NPWT device 101. This is beneficial as the user may decide to disconnect the dressing 104 from the device 101 when he/she is going to take a shower or for some other reason. It also allows a caregiver to switch the dressing from a non-absorbent type to an absorbent type depending on the status of the wound.

In figure 1, the first tubing 106 is a double conduit, whereas the second tubing 108 between the NPWT device 101 and the connector unit 110 is a single conduit. The NPWT system is by no means limited to such a construction, but may comprise a single conduit or a double conduit between the NPWT device 101 and the dressing 104.

Preferably, at least the first tubing 106 comprises a fluid conduit 111 configured to remove fluid from the dressing 104 and an air conduit 112 configured to supply air to the dressing 104, and/or the fluid conduit 111.

The NPWT system 100 comprises means to supply air to the dressing 104 at a rate of from 2 to 7 ml/min, e.g. at a rate of from 3 to 5 ml/min at a negative pressure of from - -80 to -180 mmHg, preferably from -100 to -150 mmHg, more preferably from -110 to -140 mmHg.

In the NPWT system 100 illustrated in figure 1, ambient air is introduced into the system by means of the connector unit 110 (illustrated by the arrows 113). For example, the first and/or the second connector portion (107 and 109) comprises an air filter (not shown) configured to control the supply of air into the dressing 104 and/or into the first tubing 106. The air filter may e.g. be arranged in an air inlet port of the first and/or second connector portions (107 and 109).

The air filter preferably comprises a hydrophobic and porous material, wherein the size of the pores is within the range of from 2 to 20 µm, preferably in the range of from 5 to 12 µm.The pore size of the filter is measured in a non-compressed state.

The air filter preferably comprises polyethylene, preferably sintered polyethylene.

A sintered polyethylene filter has a repeating linear molecular structure -CH2-CH2. The structure is inert with strong molecular bonds, and is characterized by improved chemical resistance, light weight, thermoplasticity and good filtering properties. A sintered polyethylene filter is also environmentally friendly as it produces no toxic waste and can be washed off and re-used.

If the means to supply air comprises an air filter, the air filter secures that the supply of air is in the range of from 2 to 7 ml/min, preferably 3 to 5 ml/min during operation, e.g. at a negative pressure of - 80 mmHg to -150 mm Hg, e.g. from -100 mmHg to -130 mmHg.

It should be noted that air may be introduced into the system in alternative ways, and an air filter may be provided at alternative positions in the system. The regulation of air supply may, in embodiments, be controlled by the NPWT device 101.

During use, the dressing 104 is arranged at a wound site of the user/patient 114, forming a sealed space. The tubing assembly 105 is configured to fluidly connect the dressing 104 to the NPWT device 101. The NPWT device 101 is then activated, e.g. by the user/patient, by pressing the start/pause button 115. The negative pressure pump is thereby activated. When activated, the negative pressure pump will start to evacuate air through the canister 103, the tubing assembly 105 (comprising the first 106 and the second tubing 108) and the sealed space formed by the dressing 104. Accordingly, the negative pressure will be created within the sealed space. In case a liquid has been formed at the wound site, this liquid may at least partly be "drawn" from the wound site, through the tubing assembly 105 and into the canister 103. The amount of liquid; i.e. exudate that is drawn from the wound and collected in the canister 103 will depend on the type of wound that is being treated as well as the type of dressing used. If the dressing 104 is absorbent, a substantially equal balance between liquid distribution is desired.

A suitable filter member (not shown) may be arranged between the canister 103 and the negative pressure pump to ensure that no liquid is allowed to pass to the negative pressure pump from the canister 103.

The NPWT system of the present disclosure may be provided as a kit of components, as illustrated in figure 2, and figure 3, respectively.

In figure 2, a kit comprising an absorbent dressing is illustrated. The kit 200 comprises at least one absorbent dressing 104 as described hereinbefore. The dressing 104 comprises a pre-attached first tubing 106. The first tubing 106 is attached by means of a coupling member 116 attached to the backing layer 117 of the dressing 104. The fact that the first tubing 106 is pre-attached allows for a quick assembly of the components of the system/kit.

The distal end of the first tubing 106 is attached to a first connector portion 107. The kit 200 further comprises a negative pressure pump arranged within a housing 102. The kit also comprises a canister 103. The canister 103 comprises a second tubing 108. The distal end of the second tubing 108 is attached to a second connector portion 109. The second connector portion 109 is configured to be connected to the first connector portion 107 associated with the first tubing 106 of the dressing 104. The kit 200 may comprise additional components such as additional batteries 118 for powering the NPWT device 101 and adhesive strips 119 for improving the adhesion between the border portion of the dressing 104 to the skin of a wearer.

The kit illustrated in figure 2 is particularly suitable for home care, but is also advantageously used in a hospital or a care facility setting. The NPWT device 101 is adapted to be carried by the user, e.g. in a pocket, belt, strap or similar. The dressing 104 and the other components of the kit 200 can easily be assembled by a user.

The components of the kit 200 may vary. For example, one kit may comprise all the components mentioned above, whereas others contain only two or three components. At least the dressing 104, the NPWT device 101 and the tubing assembly are comprised in one kit. The kit 200 may comprise a plurality of NPWT dressings as described herein before, optionally packaged together with a plurality of adhesive strips 119.

In figure 3, a kit 300 comprising a non-absorbent dressing 104 is illustrated.

In such a kit, the first tubing 106 is not pre-attached to the dressing 104, but packaged as a separated component. The first tubing 106 comprises a first distal end attached to the coupling member 116 and a second distal end attached to the first connector portion 107. The caregiver may therefore attach the coupling member 116 to the backing layer of the dressing 104, and then connect the first connector portion 107 to the second connector portion 109 of the canister tubing 108.

Furthermore, the kit 300 may comprise a wound filler material such as a gauze or a foam. In figure 3, the wound filler material is a foam 120. The wound filler material is to be applied (by a caregiver) to the wound prior to application of the dressing 104.

In embodiments, the kit comprises both an absorbent dressing and a non-absorbent dressing. This way, the caregiver can choose, depending on the characteristics of the wound, which dressing (absorbent or non-absorbent) is most appropriate to utilize. Furthermore, the caregiver can easily switch dressings if the characteristics of the wound changes.

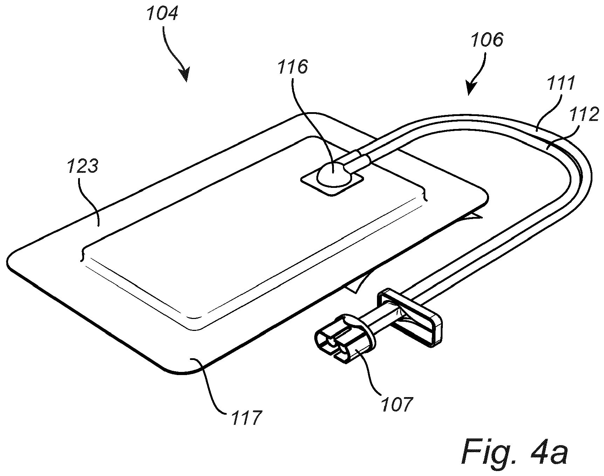

Figures 4a-c illustrate a negative pressure wound therapy (NPWT) dressing 104 in accordance with an exemplary embodiment of the present disclosure. The illustrated dressing is absorbent and suitable for use with an NPWT system of the present disclosure.

However, some features of the dressing are the same, regardless of the dressing being absorbent or non absorbent.

For example, the absorbent or non-absorbent dressing comprises a backing layer 117 comprising a thermoplastic elastomer and an adhesive skin contact layer 121 comprising a silicone gel.

The backing layer 117 is the outermost layer of the dressing 104 and is configured to face away from the skin of a wearer. The backing layer comprises a thermoplastic elastomer, which has the ability to be stretched to moderate elongations, and upon the removal of stress, return to its original shape. Examples of suitable materials comprising thermoplastic elastomer include polyurethane, polyamide and polyethylene. The backing layer may also be a laminate of polyester based nonwoven materials and at least one polyurethane film. Preferably, the backing layer comprises a thermoplastic polyurethane (TPU).

The thickness of the backing layer may be in the range of from 10 to 40 µm, preferably from 15 to 25 µm. The backing layer 117 may comprise at least one film. For example, the backing layer is a laminate formed by two or more films. A thin layer of adhesive, such as a polyacrylate adhesive, may be applied to the backing layer to attach the backing layer to the adhesive skin contact layer or, where present, an absorbent structure or any other layer of the dressing. Within the context of the present disclosure, the backing layer 117 comprises the at least one film of thermoplastic elastomer and an adhesive (e.g. polyacrylate) applied thereon. The adhesive may be applied in a continuous or discontinuous pattern.

The adhesive skin contact layer 121 is the lowermost layer of the dressing. The adhesive skin contact layer 121 is configured to detachably adhere the dressing to a dermal surface, such as the skin or the wound of a patient. In other words, the adhesive skin contact layer may also be referred to as a "wound contact layer".

The adhesive skin contact layer 121 preferably comprises a silicone gel. An adhesive skin contact layer comprising a silicone gel is skin-friendly and easy to remove without causing trauma.

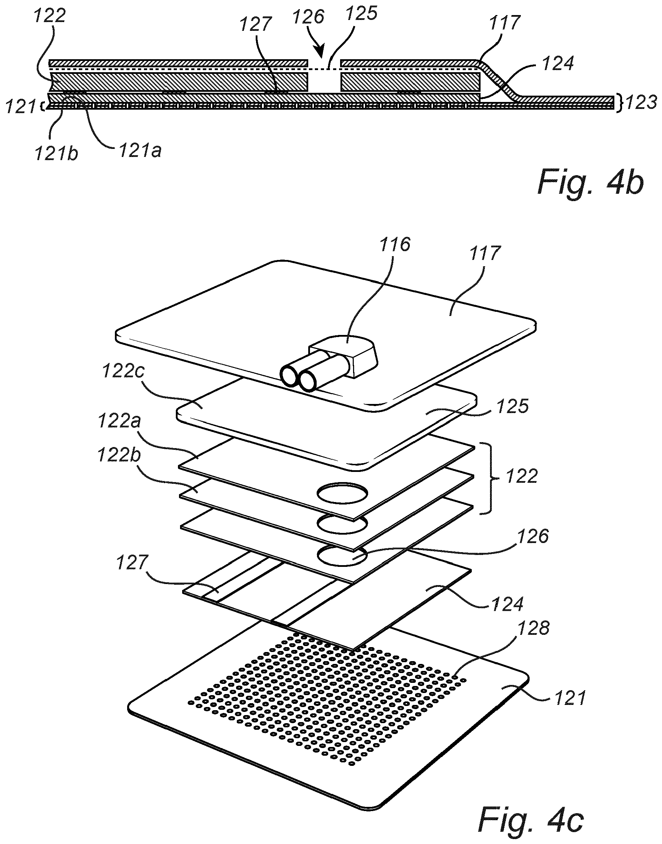

The adhesive skin contact layer 121 may be a laminate comprising a polymer based film 121a and a silicone gel layer 121b, as best illustrated in figure 4b.

The silicone gel layer 121b is configured to contact the skin of a wearer.

The polymer based film 121a is preferably a breathable film and may comprise e.g. polyethylene, polyamide or polyester polyurethane. Preferably, the polymer based film comprises polyurethane. The thickness of the polyurethane film may be from 15 to 100 µm, e.g. from 20 to 80 µm, preferably from 20 to 60 µm.

Examples of suitable silicone gels for use in the adhesive skin contact layer 121 and/or in the silicone gel layer 121b include the two component RTV systems, such as Q72218 (Dow Corning), and SilGel 612 (Wacker Chemie AG) mentioned herein, as well as NuSil silicone elastomers. In embodiments of the invention the adhesive may comprise a soft silicone gel having a softness (penetration) of from 8 to 22 mm, e.g. from 12 to 17 mm, as measured by a method based on ASTM D 937 and DIN 51580, the method being described in European Patent Application No 14194054.4. The thickness of the adhesive skin contact layer is typically at least 20 µm. The thickness of the adhesive skin contact layer may be from 100 to 200 µm.

The backing layer 117 of the absorbent or non absorbent dressing may have a moisture vapor transmission rate (MVTR) in the range of from 500 to 3500 g/m2/24h, preferably in the range of from 700 to 2600 g/m2/24h.

The "moisture vapor transmission rate (MVTR)" is the rate at which the backing layer allows moisture to permeate from the backing layer. The moisture vapor transmission rate is measured by the standard method NWSP070.4R0(15). The MVTR is measured at a temperature of 38°C.

It is generally known that exuding wounds require absorbent dressings having a significantly high moisture vapor transmission rate (MVTR). In the context of the present disclosure, a backing layer having a reduced MVTR is surprisingly associated with positive effects when the dressing is applied in negative wound therapy. A backing layer having an MVTR in the range of from 500 to 3500 g/m2/24h, preferably in the range of from 700 to 2600 g/m2/24h improves the stability of the negative pressure therapy and system, and has a positive effect on the negative pressure source; i.e. the pump, which does not need to work as hard during therapy. A more stable therapy with less frequent activations of the negative pressure source is observed, and yet, the exudate fluid collected within the dressing can be successfully evaporated from the backing layer to the surrounding. Overall, this has positive effects in terms of battery consumption, reduction of noise and a prolonged and more stable wound therapy.

With the dressing 104 of the present disclosure, a reduction in the number of pump activations by at least 26% has been observed, as demonstrated in Example 1 hereinafter.

The tensile strength of the backing layer in the machine direction (MD) and/or in the cross-machine direction (CD) may be in the range of from 30 to 70 MPa, e.g. from 35 to 55 MPa, as measured by ISO 527-3/2/200. The tensile strength is measured with 15 mm wide strips. The backing layer 117 should have a sufficient "strength" to withstand the forces inflicted on the backing layer during movement of the patient, yet allow for pliability and a sufficient degree of stretchability. The tensile strength of the backing layer also has an impact in providing a stable and reliable therapy. The backing layer should be rigid enough to prevent tearing or rupture of the backing layer during movement of the patient. For instance, the edges of the absorbent structure, where present, may be particularly vulnerable to rupture since the thicker absorbent structure may chafe against the backing layer at the edges. If perforations or slits are formed in the backing layer, this may be associated with an undesirable air leak into the dressing and the system. Consequently, the stability of the therapy and the system is impaired.

If the dressing is absorbent, the first tubing 106 may be pre-attached to the dressing 104 as illustrated in figure 4a. The first tubing 106 is attached by means of a coupling member 116 attached to the backing layer 117. The fact that the first tubing is pre-attached allows for a quick assembly of the components of the system.

The tubing 106 and/or the coupling member 116 may be of any suitable flexible material fabricated from elastomeric and/or polymeric materials. The first tubing 106 is fixedly attached to the coupling member 116. In alternative embodiments, the first tubing 106 is detachably attached to the coupling member 116.

The absorbent dressing 104 illustrated in figures 4a-c comprises a backing layer 117, an adhesive skin contact layer 121 and an absorbent structure 122 arranged between the adhesive skin contact layer 121 and the backing layer 117, wherein the backing layer 117 and the adhesive skin contact layer 121 are configured to extend beyond the periphery of the absorbent structure 122 to form a border portion 123 along the contour of the absorbent structure 122.

The absorbent structure 122 may also be referred to as a "wound pad" The wound pad may comprise additional layers apart from the absorbent structure 122. The absorbent dressing is typically referred to as "bordered dressing".

With reference to figures 4b and 4c, the absorbent dressing comprises a transmission layer 124 arranged between the adhesive skin contact layer 121 and the absorbent structure 122.

The transmission layer 124 may comprise a foam, a needled nonwoven, a through air bonded nonwoven or a spacer fabric. The transmission layer 124 is not limited to a particular material, but any material configured to ensure that negative pressure can be transmitted to the wound area during both wet and dry conditions can be used. The transmission layer 124 secures that fluid can be transported away from the wound site into the absorbent structure such that the skin can remain relatively dry.

Preferably, the transmission layer 124 comprises a spacer fabric. The spacer fabric is a three dimensional material that is often utilized in negative pressure wound therapy (NPWT) dressings.

In embodiments, the spacer fabric layer has a thickness of from 1.5 to 4 mm, e.g. from 2 to 3 mm. The basis weight of the spacer fabric may be from 150 to 500 gsm, e.g. from 200 to 350 gsm.

The spacer fabric layer 124 typically comprises a top layer and a bottom layer and an interconnecting layer of pile filaments between the top layer and the bottom layer. The interconnecting layer of pile filaments may have a fineness of 200 to 500 denier, e.g. from 250 to 350 denier.

The spacer fabric layer 124 is resistant to compression and is configured to withstand pressures exerted on the dressing during use. After a compressive force has been exerted to the dressing, the transmission layer 124 is configured to return to its original shape immediately after removal of the force.

The absorbent dressing 104 may comprise a liquid spreading layer 125 between the absorbent structure 122 and the backing layer 117.

The liquid spreading layer 125 is configured to improve the spreading of wound exudate and to create a larger surface area from which moisture can evaporate through the backing layer 117. The provision of a liquid spreading layer below the backing layer may "compensate" for the reduced moisture vapor transmission rate (MVTR) of the backing layer.

The liquid spreading layer 125 is preferably a hydrophilic and porous layer. This way, exudate can efficiently be transferred from the wound site, through the liquid spreading layer 125 to the canister (by means of the tubing assembly).

The liquid spreading layer 125 may be a fibrous material. For example, the liquid spreading layer 125 may comprise a nonwoven. A nonwoven imparts an appropriately balanced rigidity to the layer and to the dressing as such. A nonwoven liquid spreading layer 125 has the ability to distribute fluid throughout the majority of the material and to transfer the exudate in a controlled manner to the canister by means of the tubing assembly.

The liquid spreading layer 125 is also beneficial to spread potential exudate flowing from the tubing assembly 105 towards the dressing; i.e. exudate flowing in the "wrong" direction. Back-flow of exudate may occur if the person wearing the dressing disconnects the dressing from the negative pressure source and fluid connecting means. For example, the patient may disconnect the NPWT dressing if he/she is to take a shower or change clothes. The liquid spreading layer 125 secures that such back-flow of exudate is spread out rather than flowing back towards the wound site in one spot. Accordingly, the wound site can be kept relatively dry.

The liquid spreading layer 125 may comprise a meltblown, spunbond, spunlaced or carbon nonwoven. Examples of suitable polymers for use in the nonwoven are polyethylene, polyesters, polypropylene and other polyolefin homopolymers and copolymers. For example, nonwoven webs comprising thermoplastic fibres of polypropylene and polyethylene fibres or mixtures thereof may be used. The webs may have a high content of thermoplastic fibres and contain at least 50%, e.g. at least 70% thermoplastic fibres. The nonwoven may be a mixture of polyester and viscose, e.g. in a 70:30 ratio. The basis weight of the nonwoven may be in the range of from 10 to 80 g/m2, e.g. of from 20 to 50 g/m2. The liquid spreading layer may also be a spunbond- meltblown or spunbond-meltblown-spunbond (SMS) web.

The liquid spreading layer 125 preferably has the capacity to absorb wound exudate flowing from the absorbent structure. In embodiments, the liquid spreading layer 125 has an absorption capacity of at least 10g/g, as measured by the standard test method NWSP 10.1.

The absorbent structure 122 is configured to absorb wound exudate and to distribute such wound exudate in an efficient manner. As mentioned hereinbefore, if the NPWT system of the present disclosure comprises a canister and an adhesive dressing, a balanced distribution of liquid between these fluid collection means is desired. The absorbent structure 122 should thus function as a temporary reservoir to retain and distribute exudate, while also controlling the liquid transport to a canister by means of the tubing assembly.

If the dressing absorbs wound exudate too quickly and too "much", less exudate is transferred to the canister. In this situation, the dressing serves as the predominant means for fluid collection, which has the consequence that the wear time of the dressing can be impaired. In contrast, if too much exudate is transferred to the canister, then the canister may need to be emptied and replaced too often.

In embodiments, the absorbent dressing has a retention capacity of from 300 to 700 mg/cm2, preferably from 400 to 600 mg/cm2, as measured by the test method described in Example 3.

The inventors have found that the retention capacity of the dressing is important to secure that a balanced distribution of liquid between the two fluid collection means (the dressing and the canister) is achieved. Thereby, the wear time of the dressing is improved.

The dressing of the present disclosure is configured to store of from 35 to 65%, such as from 40 to 60 % of wound exudate and to remove from 35 to 65%, e.g. from 40 to 60% of the wound exudate from the dressing to the canister.

The distribution of liquid between the dressing and the canister is preferably of from 40:60 to 60:40. The inventors have found that such distribution may be maintained for up to 9 days of therapy without needing to replace the dressing (see Example 2).

The absorbent structure 122 may comprise one or a plurality of layers, wherein at least one of the layers is a superabsorbent layer 122a comprising superabsorbent polymers (SAP).

A "superabsorbent polymer" or "SAP" is a polymer that can absorb up to 300 times its own weight in aqueous fluids. Superabsorbent polymers are constituted by water-swellable and water insoluble polymers capable of absorbing large quantities of fluid upon formation of a hydrogel. The superabsorbent polymers for use in accordance with the present disclosure may be inorganic or organic crosslinked hydrophilic polymers, such as polyvinyl alcohols, polyethylene oxides, crosslinked polyacrylates and the like. Typically, the superabsorbent (SAP) comprise sodium acrylate. The SAP material may be in the form of particles, fibers, flakes or similar. Preferably, the SAP material is in the form of superabsorbent polymer (SAP) particles. The size of the superabsorbent particles may be in the range of from 45 to 850 µm, preferably from 150 to 600 µm .

In embodiments, the amount of superabsorbent particles in the absorbent structure is from 10 to 20 mg/cm2, preferably of from 13 to 17 mg/cm2.

The inventors have found that this range is beneficial for a dressing according to the present disclosure. Such an absorbent structure absorbs exudate at a "reasonable" level. If too much SAP is included, the SAP layer may swell and absorb too much and too quickly. This may have the effect that the dressing serves as the sole or at least predominant means for fluid collection. In the context of the present disclosure, the balance between the remotely arranged fluid collection means, e.g. the canister and the dressing (which is also regarded as a fluid collection means) is preferably 50:50, e.g. at least 40:60 or 60:40. As mentioned hereinbefore, this balance is important to improve the wear time of the dressing.

The absorbent structure 122 preferably has a basis weight of from 250 to 550 g/m2, preferably of from 350 to 450 g/m2.This way, the liquid distribution is controlled and a proper balance between liquid absorption and liquid removal from the dressing is observed. Furthermore, the dressing is pliable and may adapt to the movement of a wearer in a better way.

As illustrated in figure 4c, the absorbent structure 122 comprises three layers 122a-c.

The lowermost layer 122b of the absorbent structure 122 is a liquid spreading layer 122b. Exudate entering the liquid spreading layer 122b from the wound site is evenly distributed before entering the other layer(s) of the absorbent structure 122, thereby creating a larger surface area towards the superabsorbent layer 122a and other layer(s) of the absorbent structure 122.

The absorbent structure 122 may comprise a first liquid spreading layer 122b, a superabsorbent layer 122a, and a second liquid spreading layer 122c, wherein the superabsorbent layer 122a is arranged between the first and the second liquid spreading layers (122b, 122c).

The first and/or second liquid spreading layer may comprise any material having the ability to distribute the exudate in an efficient manner. For example, the first and/or second liquid spreading layer comprises a nonwoven material.

In embodiments, the first liquid spreading layer 122b is arranged below the superabsorbent layer 122a and has a greater liquid spreading capacity than the second liquid spreading layer 122c. An absorbent structure with a liquid spreading gradient is thus achieved, which impacts the ability of the absorbent structure 122 to retain, and remove, respectively, liquid from and within the dressing.

For example, the first liquid spreading layer 122b may comprise a nonwoven. The nonwoven may have a grammage in the range of from 20 to 50 gsm, e.g. from 30 to 40 gsm. The thickness of the liquid spreading layer 122b may be from 0.2 to 1.2 mm, e.g. from 0.2 to 0.6 mm.

The second liquid spreading layer 122c may be a tissue or a nonwoven layer. Typically, the spreading capability of the upper layer 122c is lower than the spreading capability of the lower liquid spreading layer 122b.

The layer 122c also serves to prevent leakage of SAP particles from the superabsorbent layer 122a. The SAP particles of the superabsorbent layer 122a chemically bind exudate entering the superabsorbent layer 122a, and thereby forms an aqueous gel. The layer 122c prevents gelling particles from moving towards the backing layer 117 and towards the coupling member 116 comprising the first tubing 106. Undesirable blockage of gel particles within the first tubing 106 is thereby prevented. The layer 122c (or 122b) may also act as a "support layer" serving as a carrier during the manufacturing process. Preferably, the layer 122c is a liquid spreading layer and serves to create a larger indirect surface of distributed liquid towards the backing layer 117 of the dressing 104.

The various layers of the absorbent structure create a complex liquid absorption and retention structure and an improved liquid distribution is observed. Particularly a controlled distribution of exudate being retained, and removed, respectively, has been observed.

In embodiments, the absorbent structure 122 comprises additional layers.

The absorbent structure 122 is preferably embossed. In other words, the surface(s) of the absorbent structure 122 is structured and may comprise a plurality of indentations and elevations (not shown). This is beneficial since an absorbent structure 122 comprising a plurality of layers may become stiff and thick as the basis weight increases. The embossing allows the absorbent structure to retain its shape and thinness, while being pliable. The embossed absorbent structure also secures a controlled spreading of wound exudate within the dressing 104. An enhanced spreading and distribution is obtained in the compressed areas of the structure.

The superabsorbent layer 122a may be an airlaid superabsorbent layer. In embodiments, the airlaid superabsorbent layer 122a comprises superabsorbent particles, cellulosic fibers and bicomponent fibers.

For example, the airlaid superabsorbent layer may comprise:
- 30-50 %, preferably 35-50 % by weight of superabsorbent particles
- 30-50 %, preferably 40-50 % by weight of cellulosic fibers
- 3-10%, preferably 5-8 % by weight of bicomponent fibers
- 3-8% by weight of polyethylene.

Such a superabsorbent layer allows for improved liquid handling properties and a proper distribution of liquid. Furthermore, it prevents gel blocking and prevents the absorbent structure from collapsing when a large amount of fluid is handled.

The bicomponent fibers act as a bonding agent, providing integrity to the SAP layer, especially in the wet state. The biocomponent fibers may be made of polyethylene and polyethylene terephthalate (PE/PET).

The thickness of the superabsorbent layer 122a may be from 0.8 to 2.5 mm, e.g. from 1.4 to 2.2 mm, e.g. from 1.8 to 2.0 mm.

As illustrated in figures 4a and 4c, the backing layer comprises a coupling member 116 configured to connect the dressing to the first tubing 106. The backing layer 117 and at least a portion of the absorbent structure 122 comprises an opening 126 (see figures 4b and 4c); the opening 126 being arranged underneath the coupling member 116, wherein the liquid spreading layer 125, if present, is void of an opening.

The opening 126 ensures fluid communication between the wound site and the canister/tubing assembly. It also enables transmission of negative pressure to the wound site. The coupling member 116 overlies the opening 126 in the backing layer (as best illustrated in figure 4c).

In figure 4c, the absorbent structure 122 comprises three layers, each of which comprises an opening 126. It is however also conceivable that an opening is provided in only one or in two layers of the absorbent structure 122.

The fact that the liquid spreading layer 125 does not contain any opening prevents gelling particles and undesired larger particulate from entering the first tubing 106 of the dressing 104.

In figure 4c, a plurality of adhesive stripes 127 are provided between the transmission layer 124 and the absorbent structure 122.

The adhesive stripes 127 are configured to halt the flow of exudate towards the coupling member 116 and the first tubing 106. As mentioned hereinbefore, the dressing 104 of the present disclosure preferably has a construction that enables a proper, and substantially equal balance between the dressing and the canister. Preferably, about 40-60% of the wound exudate is handled by the dressing, whereas 40-60% is transported to the canister.

When wound exudate is discharged from the wound, it is first handled by the transmission layer 124, and upon exit from the transmission layer 124, the adhesive stripes 127 serve to direct the exudate into the overlying absorbent structure 122, rather than flowing directly towards the tubing 106. The provision of the adhesive stripes 127 therefore contributes to the desired distribution of wound exudate between the dressing and the remotely arranged canister. The area circumventing the opening 126 is preferably free from any adhesive stripes. This is to prevent clogging and obstruction of the first tubing 106 and the coupling member 116.

A "plurality of stripes" means that the dressing comprises at least two adhesive stripes. For example, the dressing may comprise from 2 to 10, e.g. from 2 to 6 adhesive stripes depending on the size of the dressing as well as the width of the stripes.

The adhesive stripes 127 may be arranged across the width of the dressing 104. The adhesive stripes 127 may thus be arranged to extend between the lateral edges of the transmission layer 124 and/or the absorbent structure 122. The stripes are preferably arranged orthogonal to the flow path of exudate towards the tubing. Accordingly, the adhesive stripes 127 are arranged such that exudate flowing into the dressing must always meet an adhesive stripe 127 when flowing towards the tubing.

The adhesive is preferably a hot-melt adhesive. The width of the adhesive stripes may be in the range of from 3 to 25 mm, e.g. from 5 to 15 mm, e.g. from 6 to 10 mm.

The distance between the adhesive stripes 127 may be from 10 to 50 mm, e.g. from 15 to 30 mm. The distance between the adhesive stripes 127 may depend on the size and shape of the dressing 104.

As further illustrated in figure 4c, the adhesive skin contact layer 121 comprises a plurality of apertures 128 in the area underlying the absorbent structure 122, and where present, the transmission layer 124, but is void of apertures in the area forming the border portion 123.

The lack of apertures in the border portion of the dressing is beneficial to improve the adhesion at the border portion 123 of the dressing and thereby improve the stay-on ability of the dressing.

The apertures 128 extend through the polymer based film 121a (if present) and the silicone gel layer 121b. The apertures 128 improve the absorption of body fluids into the dressing 104 without compromising the adhesiveness to the skin area.

### Examples

### Example 1: System stability comparative tests

Wear tests were carried out utilizing an absorbent dressing according to an exemplary embodiment of the present disclosure (Dressing A) and a reference dressing (Dressing B). Dressing A and Dressing B were similar in construction, and differed only with respect to the backing layer. The dressings comprised, from bottom-to-top, an adhesive layer comprising a polyurethane film and a silicone gel layer, a spacer fabric transmission layer, an absorbent structure (comprising a nonwoven liquid spreading layer, an airlaid SAP layer as described hereinbefore and a tissue layer), a nonwoven liquid spreading layer and a backing layer, respectively. Both dressings comprised a pre-attached tubing comprising an air conduit and a fluid conduit. The properties of the backing layer are listed in table 1 below.

**Table 1: Backing layer material properties**

| | **Dressing A** | **Dressing B** |
|---|---|---|
| Material | Polyurethane film | Polyurethane film |
| Thickness | 20 µm | 20 µm |
| MVTR | 2530 g/m²/24h | 3940 g/m²/24h |
| Tensile strength (MD) | 39 MPa/25 mm | 24 MPa/25 mm |
| Tensile strength (CD) | 37 MPa/25 mm | 24 MPa/25 mm |

The dressings were applied to the front knees of test subjects with the leg being bent at 120 degrees (the dressing tubing pointing upwards). The tubing was connected to a mobile negative pressure device by means of a respective connector portion as illustrated in figure 1. The pump used was pump of diaphragmatic type. A canister configured to store 50 ml of liquid, as disclosed in figure 1 was connected to the pump. The connector portion attached to the distal end of the dressing tubing comprised an air filter and ambient air was introduced into the connector such that the supply of air to the dressing (by means of the air conduit) was within the range of 2-7 ml/min during operation.

The pump was activated, and a negative pressure of - 125 mmHg was applied to the dressings. The time between pump activations, Toff, was registered during the first five hours (0-5 hours, and 3-5 hours, respectively), which is an indication of the stability of the system and a means to secure that undesired air has not been introduced into the system.

Tests were performed on 5 subjects and the average Toff during time 0-5 hours, and 3-5 hours, was recorded.

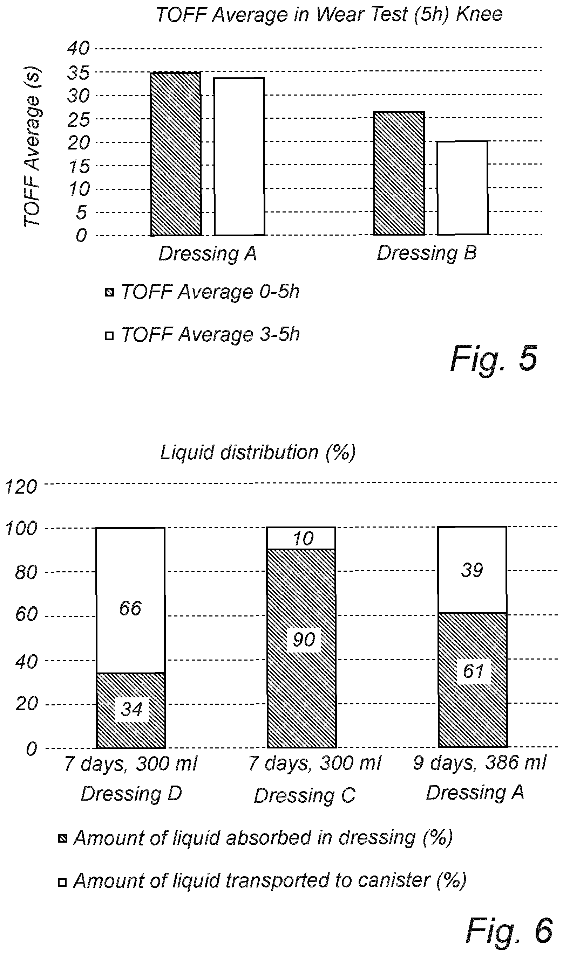

The average Toff for dressing A was 35 seconds during time 0-5 hours compared to 26 seconds for dressing B, which is an improvement of 26%. The improvement was even more significant for the time 3-5 hours, where Toff was 40% higher for the dressing of the present disclosure. The results are illustrated in figure 5 and in table 2 below. These results indicate that properties of the backing layer have an impact on the stability of the negative pressure wound therapy. The system is stable and air-tight, and the pump does not need to work as hard.

**Table 2: Average Toff comparison**

| | Dressing A | Dressing B |
|---|---|---|
| Toff average 0-5 hours | 35 s | 26 s |
| Toff average 3-5 hours | 34 s | 20 s |

### Example 2: Liquid distribution comparative tests

In order to test the distribution of liquid between the dressing and a canister, comparative tests were performed with an absorbent dressing according to an exemplary embodiment of the present disclosure (Dressing A) and two reference dressings (Dressings C and D).

Dressing A had the same construction as described hereinbefore. Dressing C had the same layer construction as Dressing A, but the basis weight of the absorbent structure was higher, and the retention capacity and amount of superabsorbent particles per cm2 was different.

Dressing D had the same general layer construction, but differed with respect to the absorbent structure. The absorbent structure of Dressing D comprised an absorbent layer comprising 40 % by weight of superabsorbent fibers (SAF) and 60 % by weight of polyester (polyethylene terephthalate) fibers as well as a nonwoven spreading layer. No superabsorbent particles were present in the absorbent structure of Dressing D.

All dressings (A, C and D) comprised a nonwoven liquid spreading layer arranged on top of the absorbent structure. The nonwoven liquid spreading layer comprised 50% by weight of viscose fibers and 50 % by weight of bicomponent fibers . See table 3 below for more details on the dressings' absorbent structures.

**Table 3: Absorption structure comparison**

| | Dressing A | Dressing C | Dressing D |
|---|---|---|---|
| Spreading layers | Yes, two | Yes, two | Yes, one |
| Basis weight | 400 g/m2 | 600 g/m2 | 370 g/m2 |
| Retention capacity per cm2 dressing | 490 mg | 780 mg | 280 mg |
| Amount of SAP particles per cm2 absorbent structure | 15 mg | 24 mg | N/A |

The retention capacity was measured as described in Example 3, hereinafter.

Pre-weighed dressings were attached to a plexiglass plate of a larger size than the dressing area. The plexiglass plate had a hole for liquid inflow. The dressings were positioned so that the liquid inflow was in the middle portion of the dressing. Each dressing comprised a tubing that was connected to a mobile negative pressure device as illustrated in figure 1. The pump used was a pump of diaphragmatic type. A canister configured to store 50 ml of liquid was used and was connected to the pump arranged within a housing as disclosed in figure 1. The dressing and the NPWT device (comprising the canister and the pump) were connected by respective connector portions, as described hereinbefore. An air filter was arranged within the first connector portion associated with the dressing tubing. Ambient air was introduced into the connector such that the supply of air to the dressing was within the range of 2-7 ml/min. The pump was activated, and a negative pressure of - 125 mmHg was applied to the dressings.

Test liquid (horse serum) was added in the middle of each dressing with a flow of 300 ml in 7 days (dressing C and D), and of 386 ml in 9 days (dressing A). The negative pressure in the dressing was maintained at -125 mmHg during the whole test period. After the test period, the wet weight of the dressings and the canister was recorded. The distribution of test liquid between each dressing and canister was calculated.

As can be seen in figure 6, the liquid distribution between dressing A and the canister was 61:39, whereas for dressing C, the majority of the liquid was kept in the dressing (90%) with only 10% being transferred to the canister. Dressing D had a dressing:canister liquid distribution of 34:66.

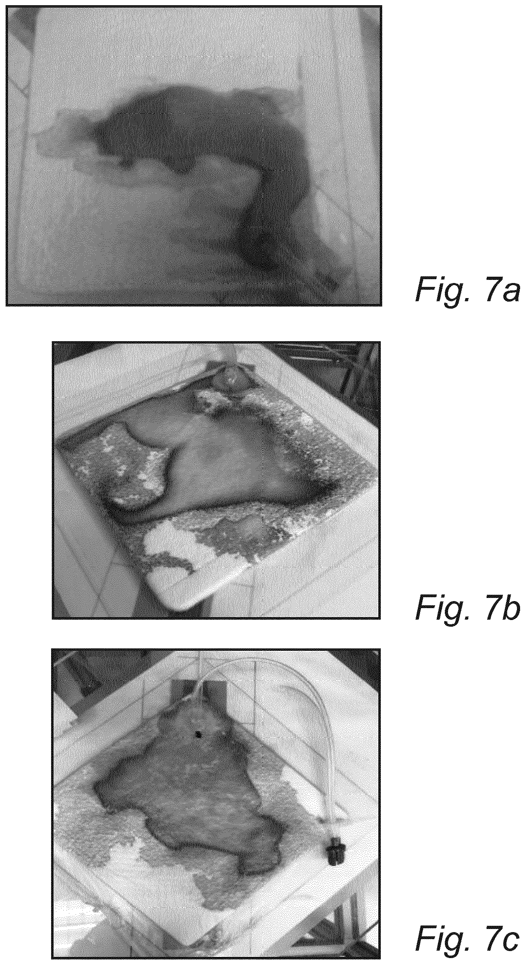

Pictures were also taken on the dressings after the test periods (7 days, and 9 days, respectively). As can be seen in figure 7a, Dressing D had a poor liquid distribution within the dressing structure. In other words, only a small proportion of the absorbent capacity of the dressing was utilized. Instead, more exudate was transferred to the canister.

Figure 7b illustrates Dressing C, where a large proportion of the dressing was utilized. Although not clearly visible from this figure, the dressing had bulky and "soaky" appearance.

Figure 7c illustrates Dressing A after 9 days of liquid exposure. A large proportion of the dressing was utilized for liquid handling, while still allowing for at least 40% of exudate to be transferred to the canister. A desired liquid distribution between the fluid collection means (dressing and canister) was thereby achieved.

### Example 3: Retention capacity of the dressing

The fluid retention capacity is defined as the capability of dressing to retain liquid.

First, the theoretical maximum absorption was evaluated for the dressing samples. The maximum absorption capacity is the amount of liquid that the dressing is able to absorb when exposed to excess test liquid and in absence of an applied load.

Dressing samples A, C and D were punched to a predefined size (5x5cm = 25cm²) from the central part of the dressing (such that all layers present in the dressing were used in the test).

The area and weight of the dressing samples (A, C and D) in a dry state were recorded. Each dressing sample was soaked in a bowl with a generous volume of test liquid (horse serum). A wire gauze was placed on top of the sample to force it down below the liquid surface, with the adhesive skin contact layer towards the wire gauze. Each sample was left to absorb for 60 minutes, covered with test liquid during the whole absorption time. When the absorption time was completed, the sample was hung vertically in one dressing corner to drain for 120 seconds. The samples were allowed to absorb liquid during 60 minutes. When the absorption time was completed, the specimens were drained freely for 120 seconds, held vertically in one corner (see figure below). The maximum absorption capacity was recorded in g liquid for each of the samples.

After the maximum absorption capacity had been calculated, a similar test was performed (as described above). The samples wer allowed to absorb test liquid corresponding to 80% of the theoretical maximum absorption. After 10 minutes absorbing time, a pressure equivalent to 125 mmHg was added to the sample, with the wound side of the sample facing down. The static pressure was remained during 120 seconds. The retention was then calculated as the weight of horse serum retained in the sample after exposure to static pressure. The retention capacity is thus the ability of a product to hold liquid under a specified amount of pressure. The retention capacity for dressings A, C and D are illustrated in table 3 hereinbefore.

### Example 4: Effect of liquid spreading layer in preventing back-flow of liquid

In order to test the ability of the dressing to handle back-flow of exudate, which may be a problem when a dressing is disconnected from the NPWT device, a comparative test was set up with an absorbent dressing according to an exemplary embodiment of the present disclosure (Dressing A as described hereinbefore), and a reference dressing (Dressing E). Dressing E had the same construction as Dressing A, but lacked a nonwoven liquid spreading layer between the backing layer and the absorbent structure. The tubing of each dressing was connected to a mobile negative pressure device by means of the same procedure as described in Examples 1-2.

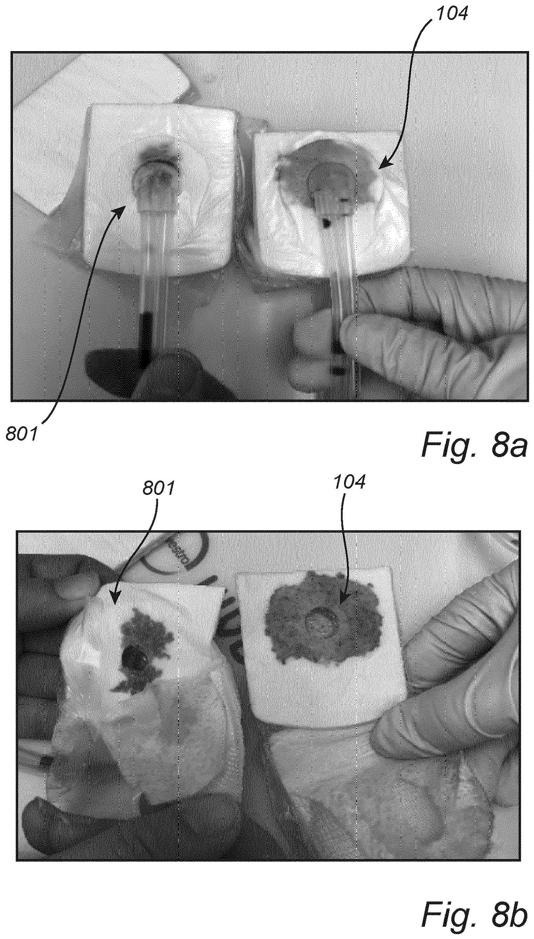

The canister was filled with approximately 52 ml horse serum (excess liquid). When the negative pressure of - 125 mmHg was stable, the canister was disconnected from the pump and the excess liquid was transported back to the dressing. As can be seen in figure 8a and 8b, the back-flow of exudate was distributed over a larger surface with a dressing of the present disclosure (Dressing A), denoted 104 in figures 8a and 8b. In contrast, the back-flow of exudate in Dressing E (denoted 801 in figures 8a and 8b), was not spread out to a significant degree, and a larger proportion of exudate was transferred directly back towards the wound site. The liquid spreading layer thereby contributes to an even exudate spreading and distribution in both directions.

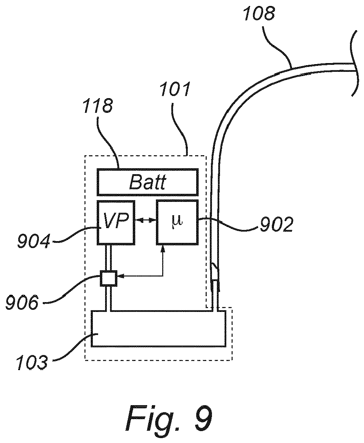

Turning finally to figure 9 present an exemplary functional scheme of a NPWT device 101 according to an exemplary embodiment of the present disclosure. In addition to the discussion provided above, figure 9 presents the NPWT device 101 to further comprise a control unit 902, electrically connected to the battery 118 and adapted to control an operation of the negative pressure pump 904. The control unit 902 may include a microprocessor, microcontroller, programmable digital signal processor or another programmable device. The control unit 902 may also, or instead, each include an application specific integrated circuit, a programmable gate array or programmable array logic, a programmable logic device, or a digital signal processor. Where the control unit 902 includes a programmable device such as the microprocessor, microcontroller or programmable digital signal processor mentioned above, the processor may further include computer executable code that controls operation of the programmable device.

In addition, the NPWT device 101 comprises at least one pressure sensor 906 arranged in fluid connection with the negative pressure pump 904. The NPWT device 101 preferably also comprises a memory element (not shown) arranged in communication with the control unit 902. The memory element preferably holds information as to a predefined supply rate as well as well as a predetermined level of negative pressure, wherein the predetermined level of negative pressure is from -80 to -180 mmHg, preferably from -100 to -150 mmHg, more preferably from -110 to -140 mmHg.

The control unit 902 will then, based on the determined intermediate voltage, the predetermined supply rate and the predetermined level of negative pressure select a suitable pulse width modulation (PWM) scheme for operating the negative pressure pump. When selecting the PWM scheme, the control unit 902 will also take into account the desire to keep an operational speed of the negative pressure pump 904 to be essentially constant, or at least within a range of +/- 10% of the desired operational range, to ensure that the end user is subjected to a minimal amount of noise disturbance.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A negative pressure wound therapy (NPWT) system (100) comprising
- a mobile negative pressure wound therapy (NPWT) device (101) comprising a housing (102), a negative pressure pump arranged within said housing (102) and a canister (103) detachably connected to said housing (102),
- an absorbent or a non-absorbent dressing (104),
- a tubing assembly (105) configured to fluidly connect said absorbent or non-absorbent dressing (104) to said NPWT device, wherein said tubing assembly (105) comprises a
- a first tubing (106) configured to be connected to said dressing (104), wherein a distal end of said first tubing (106) is attached to a first connector portion (107);
- a second tubing (108) configured to be connected to said canister (103), wherein a distal end of said second tubing (108) is attached to a second connector portion (109) configured to be detachably connected to said first connector portion (107), wherein said system (100) comprises means to supply air to said dressing (104) at a rate of from 2 to 7 ml/min during operation.

2. The negative pressure wound therapy (NPWT) system (100) of claim 1, wherein said means to supply air to said dressing is configured to supply air at a rate of from 2 to 7 ml/min, preferably from 3 to 5 ml/min, at a negative pressure of from -80 to -180 mmHg, preferably from -100 to -150 mmHg, more preferably from -110 to -140 mmHg.

3. The negative pressure wound therapy (NPWT) system (100) of claim 1 or claim 2, wherein said means to supply air comprises an air filter arranged in said first (107) or said second (109) connector portion, wherein said air filter is configured to control the supply of ambient air into said dressing (104) and/or said first tubing (106).

4. The negative pressure wound therapy (NPWT) system (100) of claim 3, wherein said air filter comprises a hydrophobic and porous material, wherein the size of the pores is within the range of from 2 to 20 µm, preferably in the range of from 5 to 12 µm.

5. The negative pressure wound therapy (NPWT) system (100) of claim 3 or claim 4, wherein said air filter comprises polyethylene, preferably sintered polyethylene.

6. The negative pressure wound therapy (NPWT) system (100) of any one of the preceding claims, wherein said first tubing (106) comprises a fluid conduit (111) configured to remove fluid from said dressing (104) and an air conduit (112) configured to supply air to said dressing (104) and/or said fluid conduit (111).

7. The negative pressure wound therapy (NPWT) system (100) of any one of the preceding claims, wherein said non-absorbent or absorbent dressing (104) comprises at least a backing layer (117) comprising a thermoplastic elastomer and an adhesive skin contact layer (121) comprising a silicone gel.

8. The negative pressure wound therapy (NPWT) system (100) of claim 7, wherein said adhesive skin contact layer (121) is a laminate comprising a polyurethane film (121a) and a silicone gel layer (121b).

9. The negative pressure wound therapy (NPWT) system (100) of any one of the preceding claims, wherein the backing layer (117) of said dressing (104) has a moisture vapor transmission rate (MVTR) in the range of from 500 to 3500 g/m2/24h, preferably in the range of from 700 to 2600 g/m2/24h as measured by NWSP070.4R0(15).

10. The negative pressure wound therapy (NPWT) system (100) of any one of the preceding claims, wherein said dressing (104) is absorbent and said first tubing (106) is pre-attached to said dressing (104).

11. The negative pressure wound therapy (NPWT) system (100) of any one of the preceding claims, wherein said dressing (104) is absorbent and comprises a backing layer (117), an adhesive skin contact layer (121) and an absorbent structure (122) arranged between said adhesive skin contact layer (121) and said backing layer (117), wherein said backing layer (117) and said adhesive skin contact layer (121) are configured to extend beyond the periphery of said absorbent structure (122) to form a border portion along the contour of said absorbent structure (122).

12. The negative pressure wound therapy (NPWT) system (100) according to claim 11, wherein said absorbent dressing (104) comprises a transmission layer (124) arranged between said adhesive skin contact layer (121) and said absorbent structure (122), wherein said transmission layer (124) comprises a spacer fabric material.

13. The negative pressure wound therapy (NPWT) system (100) according to claim 10 or claim 11, wherein said absorbent dressing (104) comprises a liquid spreading layer (125) between said absorbent structure (122) and said backing layer (117).

14. The negative pressure wound therapy (NPWT) system (100) according to any one of the preceding claims, wherein said absorbent dressing (104) has a retention capacity of from 300 to 700 mg/cm2, preferably from 400 to 600 mg/cm2, as measured by the test method described in the specification.

15. The negative pressure wound therapy (NPWT) system (100) according to anyone of claims 11 and 12, wherein said backing layer (117) comprises a coupling member (116) configured to connect the dressing (104) to said first tubing (106) and wherein said backing layer (117) and at least a portion of said absorbent structure (122) comprises an opening (126); said opening (126) being arranged underneath said coupling member (116), wherein said liquid spreading layer (125), if present, is void of an opening.

16. A negative pressure wound therapy (NPWT) system (100), the NPWT system comprising:
- a wound dressing (104), the wound dressing provided for creating a sealed space defined in part by a wound site, the wound dressing being at least one of an absorbent or a non-absorbent dressing, the non-absorbent or absorbent dressing comprises at least a backing layer (117) comprising a thermoplastic elastomer and an adhesive skin contact layer comprising a silicone gel,
- means for supplying air to the wound dressing at a predetermined supply rate, the rate being from 2 to 7 ml/min, preferably from 3 to 5 ml/min and
- an NPWT device (101), comprising:
- a housing (102),
- a battery (118) arranged within the housing,
- a negative pressure pump (904) arranged within the housing,
- a control unit (902) arranged within the housing, the control unit being electrically connected to the battery and the negative pressure pump, and
- a canister (103) fluidly coupled to the negative pressure pump and to the wound dressing,
wherein the control unit is arranged to:
- determine an intermediate voltage level of the battery,
- select a pulse width modulation (PWM) scheme for operating the negative pressure pump, the PWM scheme selected based on the predetermined supply rate, the intermediate voltage level and a predetermined level of negative pressure, wherein the predetermined level of negative pressure is from -80 to -180 mmHg, preferably from -100 to -150 mmHg, more preferably from -110 to -140 mmHg, and
- operate the negative pressure pump according to the selected PWM scheme.

17. The NPWT system (100) of any one of the preceding claims, wherein said NPWT system is provided as a kit of components and wherein said kit comprises both a non-absorbent and an absorbent dressing.
